Europäisches Patentamt
European Patent Office
Office européen des brevets

(19) (11) Numéro de publication: **0 288 360 A1**

(12) # DEMANDE DE BREVET EUROPEEN

(21) Numéro de dépôt: **88400903.6**

(22) Date de dépôt: **14.04.88**

(51) Int. Cl.⁴: **C 07 D 295/08**
C 07 D 295/10, A 61 K 31/495

(30) Priorité: **14.04.87 FR 8705311**

(43) Date de publication de la demande:
**26.10.88 Bulletin 88/43**

(84) Etats contractants désignés:
**AT BE CH DE ES FR GB GR IT LI LU NL SE**

(71) Demandeur: **Société anonyme: LES LABORATOIRES MERAM**
**4 Bld Malesherbes**
**F-75008 Paris (FR)**

(72) Inventeur: **Buzas, André**
**25, route de Versailles**
**F-91570 Bievres (FR)**

**Ollivier, Roland**
**94, rue des Fauvettes**
**F-45160 Olivet (FR)**

(74) Mandataire: **Gillard, Marie-Louise et al**
**Cabinet Beau de Loménie 55, Rue d'Amsterdam**
**F-75008 Paris (FR)**

(54) **Dérivés de la 1-[(1,1-diphényl)-1-alcényl]-pipérazine, procédé d'obtention et compositions pharmaceutiques les contenant.**

(57) L'invention concerne des dérivés de la 1-[(1,1-diphényl)-1-alcényl]-pipérazine répondant à la formule générale I :

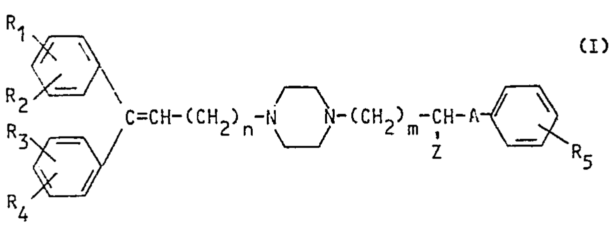

(I)

dans laquelle $R_1$, $R_2$, $R_3$, $R_4$ et $R_5$, identiques ou différents, représentent un atome d'hydrogène, un atome d'halogène, un groupe alkyle inférieur, un groupe alcényle inférieur, un groupe alcoxy inférieur ou le groupe trifluorométhyle ; n est un nombre entier compris entre 1 et 3; m est un nombre entier de 0 à 3 ; Z représente un atome d'hydrogène, un groupe alkyle inférieur, ou un groupe aryle de formule

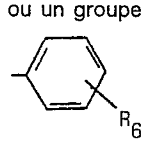

dans laquelle $R_6$ a la même signification que $R_1$, $R_2$, $R_3$ ou $R_4$ ; A est un atome d'oxygène ou un groupe $-\underset{O}{\underset{\|}{C}}-$; et leurs sels pharmaceutiquement acceptables.

Application : compositions pharmaceutiques ayant des propriétés anti-dépressives.

EP 0 288 360 A1

**Description**

**Dérivés de la 1-[(1,1-diphényl)-1-alcényl]-pipérazine, procédé d'obtention et compositions pharmaceutiques les contenant.**

La présente invention concerne des dérivés de la 1-[(1,1-diphényl)-1-alcényl]-pipérazine. Elle concerne également les sels pharmaceutiquement acceptables de ces dérivés. De plus, elle a aussi pour objet les procédés pour leur obtention et les compositions pharmaceutiques les contenant.

Les dérivés selon l'invention présentent des propriétés pharmacologiques intéressantes sur le système nerveux central et en particulier des propriétés antidépressives, mais ne possèdent pas ou peu d'activité sédative.

Les dérivés de l'invention répondent à la formule générale ci-après :

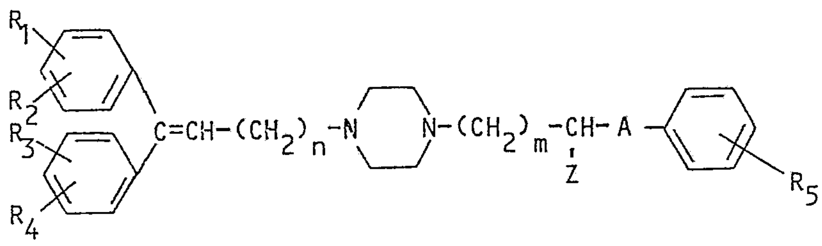

(I)

dans laquelle :

$R_1$, $R_2$, $R_3$, $R_4$ et $R_5$, identiques ou différents, représentent un atome d'hydrogène, un atome d'halogène, un groupe alkyle inférieur, un groupe alcényle inférieur, un groupe alcoxy inférieur ou le groupe trifluorométhyle;

n est un nombre entier compris entre 1 et 3;

m est un nombre entier de 0 à 3 ;

Z représente un atome d'hydrogène, un groupe alkyle inférieur ou un groupe aryle de formule

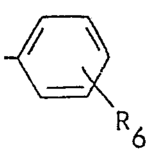

dans laquelle $R_6$ a la même signification que $R_1$, $R_2$, $R_3$ ou $R_4$;

A est un atome d'oxygène ou un groupe $-\underset{\overset{\|}{O}}{C}-$

Dans la présente description, on désigne par

- "alkyle inférieur" les restes d'hydrocarbures aliphatiques saturés à chaîne droite ou ramifiée contenant 1 à 6 atomes de carbone. Le groupe alkyle préféré aux fins de l'invention est le groupe méthyle;
- "alcényle inférieur" les restes d'hydrocarbures aliphatiques insaturés à chaîne droite ou ramifiée contenant 1 à 6 atomes de carbone;
- "alcoxy inférieur" un groupe hydroxy substitué par un alkyle inférieur tel que défini ci-dessus.

Les composés de l'invention peuvent être obtenus par réaction d'un dérivé de formule générale II :

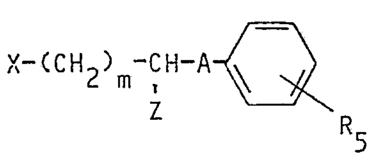

(II)

dans laquelle Z, m, A sont tels que définis ci-dessus, et X est un groupe tosyle ou un atome d'halogène (par exemple le brome ou le chlore),

avec une pipérazine substituée de formule générale III :

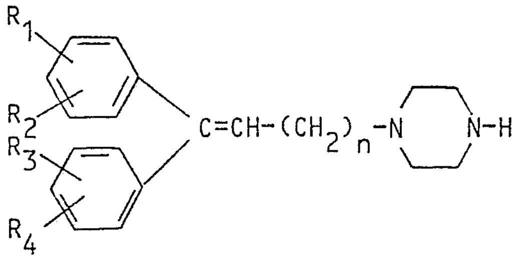

(III)

dans laquelle $R_1$, $R_2$, $R_3$, $R_4$ et n sont tels que définis précédemment, en présence d'iodure de sodium ou de potassium et d'un équivalent de carbonate de sodium ou de potassium.

Cette réaction est réalisée par chauffage dans un solvant approprié, tel qu'un hydrocarbure aromatique, par exemple le toluène ou le benzène ou dans un autre solvant, tel que la méthyléthylcétone.

Les dérivés de formule générale III peuvent aisément être obtenus par réaction d'un dérivé de formule générale IV :

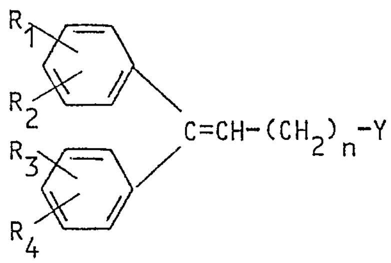

(IV)

dans laquelle $R_1$, $R_2$, $R_3$, $R_4$ et n sont tels que définis ci-dessus, et Y est un atome de brome ou de chlore, avec la pipérazine anhydre de formule V :

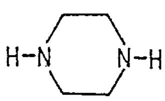

(V)

Ou utilise en général 4 équivalents du composé de formule V pour un équivalent du composé de formule IV.

Cette condensation est réalisée par chauffage à reflux dans un solvant approprié, tel qu'un hydrocarbure aromatique, par exemple le benzène ou le toluène.

Les composés de formule IV peuvent être préparés par la méthode décrite par DAVIS et al. dans J. Med. Chem. 10, (4) 627-635, 1967.

Les composés de formule II pour lesquels m est un entier égal à 2, A étant un atome d'oxygène, Z un radical aryl, X un atome de chlore, $R_1$, $R_2$, $R_3$, $R_4$ et $R_5$ étant tels que définis précédemment peuvent être également obtenus par la méthode décrite par SLIWA et al. dans "Bull. Soc. Chim. Fra., 1972, 4, 1540-1544" selon le schéma réactionnel ci-après :

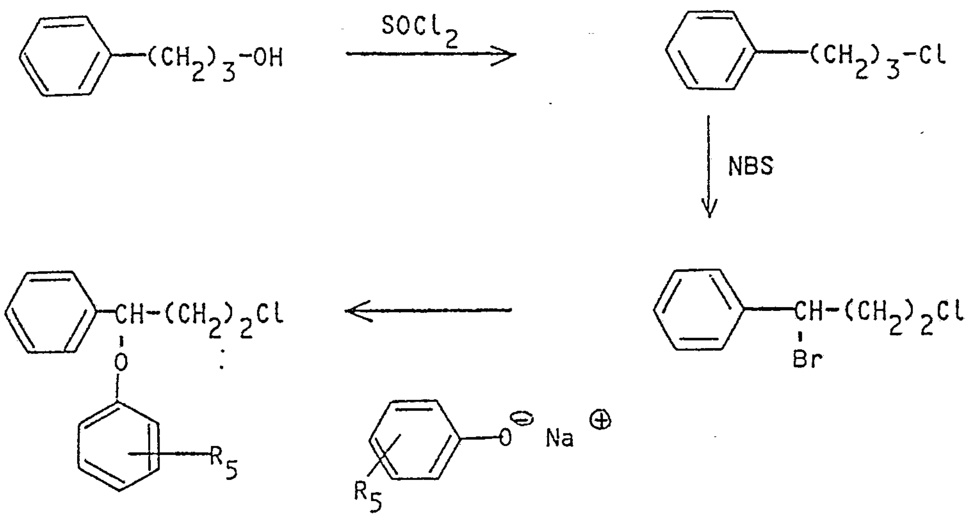


Les autres composés de formule II dans lesquels A est l'oxygène peuvent être obtenus par des méthodes classiques d'addition de phénates sur des composés dihalogénoalkylés. Certains de ces composés sont disponibles dans le commerce.

Les composés de formule II dans lesquels A est le groupe $-\underset{\underset{O}{\|}}{C}-$ peuvent être obtenus par la réaction bien connue de Friedel

et Craft. Dans les exemples ci-après, on a utilisé le chlorure de parafluorobutyrophénone disponible dans le commerce.

Les sels d'addition d'acides des dérivés de formule I selon l'invention peuvent être obtenus par des procédés classiques avec des acides couramment utilisés pour obtenir des sels pharmaceutiquement acceptables, tels l'acide chlorhydrique, l'acide méthane-sulfonique, l'acide tartrique, l'acide maléique ou l'acide fumarique.

Comme précisé précédemment, le dérivés selon l'invention possèdent des propriétés pharmacologiques intéressantes sur le système nerveux central et notamment des propriétés antidépressives.

L'invention a donc également pour objet les compositions pharmaceutiques contenant à titre de principe actif un dérivé selon l'invention en combinaison avec un véhicule pharmaceutiquement acceptable.

Les compositions selon l'invention peuvent être des compositions administrables par voie orale ou par voie injectable. Elles peuvent se présenter sous la forme de solutions, de comprimés, de gélules, de pilules ou de compositions injectables.

L'invention va être maintenant décrite plus en détail par les exemples illustratifs ci-après. Das ces exemples, les dérivés préparés ont été identifiés et caractérisés grâce à l'étude de leurs spectres RMN et infrarouge ainsi qu'à leur analyse élémentaire.

Le dérivé de pipérazine utilisé comme composé de départ a été préparé selon le mode opératoire ci-après :

Préparation de la 1-[(1,1-diphényl)-1-butène-4-yl]-pipérazine

Dans un réacteur de 250 ml, on a placé 34,4 g de pipérazine anhydre en solution dans 110 ml de toluène. On a porté le mélange à reflux et on a ajouté goutte à goutte une solution de 28,7 g de 4-bromo-1,1-diphényl-1-butène dans 15 ml de toluène. On a agité le mélange pendant 1 h.

On a refroidi la solution. Après filtration, on a repris par 100 ml d'eau et décanté. On a extrait la phase toluénique par 3 fois avec 50 ml d'une solution d'acide acétique à 7%. On a neutralisé par du carbonate de sodium en présence de 100 ml de $CH_2Cl_2$. On a décanté, séché et évaporé le solvant. On a recueilli 22,5 g d'huile brune qui a été utilisée brute dans l'exemple suivant.

Formule brute : $C_{20}H_{24}N_2O$

Spectre RMN (solvant $CDCl_3$, référence TMS) 1,2 ppm (s), 1H, NH; 2,2 ppm (m), 8H, $CH_2$-N-$CH_2$-$CH_2$-C=C; 2,7 ppm (m), 4H, H-N-$CH_2$; 5,8 ppm (t), 1H, C=CH; 7,0 ppm (M), 10H, Ø.

EXEMPLE 1 : Préparation du diméthane-sulfonate de la 1-[(1,1-diphényl)-1-butène-4-yl]-4-[1-(2-(4-fluorophénoxy)éthyl]pipérazine (1)

Dans un réacteur de 250 ml, on a placé 13,33 g de 1-(1,1-diphényl-1-butène-4-yl)-pipérazine préparée précédemment en solution dans 150 ml de méthyléthylcétone, 10 g de 2-bromo-1-(4-fluorophénoxy)-éthane, 9,45 g de $Na_2CO_3$ et 0,1 g de NaI anhydre.

On a chauffé le mélange réactionnel à reflux pendant 18 h, puis on a refroidi. Après filtration, on a lavé la

solution par 2 fois avec 80 ml d'eau.

On a décanté, séché et évaporé le solvent. On a recueilli 10,5 g d'huile jaune.

Afin de purifier le produit, on a effectué et recristallisé le chlorhydrate dans l'acétone.

A 7 g de l'huile brute obtenue précédemment, on a ajouté 100 ml d'une solution d'acide chlorhydrique 1N. On a agité le mélange 30 min. Après essorage et rinçage par 2 fois avec 50 ml d'eau, on a recristallisé le chlorhydrate obtenu dans 30 ml d'acétone.

On a libéré la base par du $Na_2CO_3$ en présence de 50 ml d'eau et 50 ml de $CH_2Cl_2$. On a décanté, séché et évaporé le solvant. On a obtenu 6,3 g d'huile.

Le diméthane-sulfonate a été préparé en faisant réagir 4 g du produit obtenu en solution dans l'éther, avec 1,8 g d'acide méthane-sulfonique. On a obtenu un solide de point de fusion F = 150°C et de formule brute : $C_{28}H_{31}FN_2O,2(CH_4O_3S)$.

Spectre RMN (solvant $CDCl_3$, référence TMS) de la base 2,5 ppm (M), 14 H, $=C-\underline{CH_2}-\underline{CH_2}-N-\underline{CH_2}-\underline{CH_2}$; 4,0 ppm (t), 2H, $O-CH_2$; 6,0 ppm (t), 1H,C=CH; 7,0 ppm (m), 14H, Ø.

Spectre IR (1% dans le KBr) 2500 $cm^{-1}$ ($N^+$-H); 1200 $cm^{-1}$ ($SO_2$); 1060 $cm^{-1}$ ($SO_2$)

EXEMPLES 2 à 17

En opérant selon le mode opératoire décrit dans l'exemple 1 ci-dessus, on a obtenu les composés n° 2 à 17 indiqués dans le tableau ci-après.

TABLEAU I

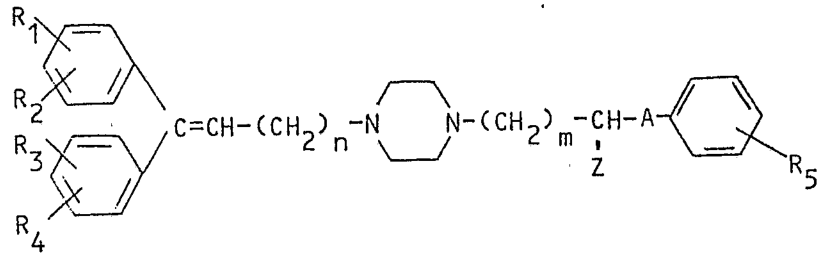

| Composé | $R_1=R_2=R_3=R_4$ | $R_5$ | Z | n | m | A | Fusion °C | Masse molaire | Sel |
|---|---|---|---|---|---|---|---|---|---|
| 1 | H | 4-F | H | 2 | 1 | 0 | 150 | 622 | Diméthane-sulfonate |
| 2 | H | $2-CH_3$ | H | 2 | 1 | 0 | 100[a] | 618 | |
| 3 | H | 3-Cl | H | 2 | 1 | 0 | 90[a] | 638,5 | |
| 4 | H | $2-CH_3$ | 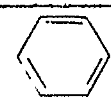 | 2 | 2 | 0 | 80[a] | 708 | |
| 5 | H | 4-F | " | 2 | 2 | 0 | 85[a] | 712 | |
| 6 | H | 4-F | H | 2 | 2 | 0 | 155 | 636 | |
| 7 | H | 4-F | H | 2 | 2 | C=0 | 140 | 648 | |
| 8 | H | 4-F | H | 2 | 3 | 0 | 134 | 650 | |
| 9 | H | H | H | 2 | 1 | 0 | 200 | 604 | |
| 10 | H | $4-t(C_4H_9)$ | H | 2 | 2 | 0 | 222 | 674 | |
| 11 | H | 4-Cl | H | 2 | 2 | 0 | 156 | 625,5 | |
| 12 | H | $3-OCH_3$ | H | 2 | 2 | 0 | 189 | 648 | |
| 13 | H | 4-F | H | 2 | 3 | 0 | 154 | 650 | |

a : Point de décomposition

TABLEAU I (suite)

| Composé | $R_1=R_2=R_3=R_4$ | $R_5$ | Z | n | m | A | Fusion °C | Masse molaire | Sel |
|---|---|---|---|---|---|---|---|---|---|
| 14 | H | $4-CH_3$ | H | 2 | 3 | 0 | 157 | 646 | Diméthane-sulfonate |
| 15 | H | $4-t(C_4H_9)$ | H | 2 | 3 | 0 | 191 | 688 | |
| 16 | H | 4-F | H | 3 | 1 | 0 | 165 | 636 | |
| 17 | $R_1=4-F, R_2R_3R_4=H$ | 4-F | H | 2 | 1 | 0 | 151 | 640 | |

Les spectres RMN de ces composés sont donnés ci-après : (solvant $CDCl_3$ - référence interne TMS).

Dérivé 2 : 2,3 ppm (s), 3H, $CH_3$; 2,5 ppm (m), 14H, $CH_2$-$CH_2$-N-$CH_2$-$CH_2$-N; 4,1 ppm (t), 2H, O-$CH_2$; 6,1 ppm (t), 1H, C=CH; 7,0 ppm (m) 14H, Ø.

Dérivé 3 : 2,5 ppm (m), 14H, $CH_2$-$CH_2$-N-$CH_2$-$CH_2$-N; 4,0 ppm (t), 2H, O-$CH_2$; 6,0 ppm (t), 1H, C=CH; 7,0 ppm (m), 14H, Ø.

Dérivé 4 : 2,3 ppm (m), 16H, $CH_2$-$CH_2$-N-$CH_2$-$CH_2$-N-$CH_2$; 2,4 ppm (s), 3H, $CH_3$; 5,1 ppm (t), 1H, CH-O; 5,9 ppm (t), 1H, C=CH; 7,1 ppm (m), 19H, Ø.

Dérivé 5 : 2,4 ppm (m), 16H, $CH_2$-$CH_2$-N-$CH_2$-$CH_2$-N-$CH_2$; 5,1 ppm (t), 1H, O-CH; 6,0 ppm (t), 1H, C=CH; 7,0 ppm (m), 19H, Ø.

Dérivé 6 : 2,0 ppm (m), 2H, C-$CH_2$-C-O; 2,3 ppm (m), 14H, $CH_2$-$CH_2$-N; 3,7 ppm (t), 2H, O-$CH_2$; 5,8 ppm (t), 1H, C=CH; 6,5 à 7,2 ppm (m), 14H, Ø.

Dérivé 7 : 1,6 à 2,6 ppm (m), 16H, $CH_2$-$CH_2$-N; 1,8 ppm (t), 2H, $CH_2$-C=O; 5,8 ppm (t), 2H, C=CH; 6,5 à 7,8 ppm (m), 14H, Ø.

Dérivé 8 : 1,8 ppm (m), 4H, N-C-$CH_2$-$CH_2$-C-O; 2,3 ppm (m), 12H, $CH_2$-N; 3,7 ppm (t), 2H, O-$CH_2$; 5,8 ppm (t), 1H, C=CH; 6,5 à 7,4 ppm (m), 14H, Ø.

Dérivé 9 : 2,4 ppm (m), 14H, $CH_2$-$CH_2$-N-$CH_2$-$CH_2$-N-$CH_2$; 4,0 ppm (t), 2H, $CH_2O$; 6,0 ppm (t), 1H, C=CH; 7,2 ppm (m), 15H, Ø.

Dérivé 10 : 1,2 ppm (s), 9H, $CH_3$; 1,9 ppm (m), 2H, C-$CH_2$-C; 2,3 ppm (m), 14H, $CH_2$-N; 4,0 ppm (t), 2H; $CH_2O$; 6,0 ppm (t), 1H, C=CH; 1,0 ppm (m), 14H, Ø.

Dérivé 11 : 1,8 ppm (m), 2H, $CH_2$; 2,4 ppm (m), 14H, $CH_2N$; 3,9 ppm (t), 2H, $CH_2O$; 6,0 ppm (t), 1H, C=CH; 7,0 ppm (m), 14H, Ø.

Dérivé 12 : 2,3 ppm (m) 16H, $CH_2$-N, $CH_2$; 3,5 ppm (s), 3H, $CH_3O$; 3,7 ppm (t), 2H, $CH_2O$; 5,9 ppm (t), 1H, C=CH; 6,7 à 7,4 ppm (m), 14H, Ø.

Dérivé 13 : 1,7 à 2,4 ppm (m), 18H, $CH_2$-N, $CH_2$; 3,8 ppm (t), $CH_2O$; 6,0 ppm (t), 1H, C=CH; 6,8 à 7,2 ppm (m), 14H, Ø.

Dérivé 14 : 1,8 ppm (m), 4H, N-C-$CH_2$-$CH_2$-C-O; 2,4 ppm (s), 3H, $CH_3$, 2,3 à 2,6 ppm (m), 14H, $CH_2$-$CH_2$-N-$CH_2$-$CH_2$-N-$CH_2$; 3,9 ppm (t), 2H, $CH_2$-O; 6,0 ppm (t), 1H, CH=C; 6,5 à 7,2 ppm (m), 14H, Ø.

Dérivé 15 : 1,2 ppm (s), 9H, $CH_3$; 1,7 ppm (m), 4H, N-C-$CH_2$-$CH_2$-C-O; 2,1 à 2,6 ppm (m), 14H, $CH_2$-$CH_2$-N-$CH_2$-$CH_2$-N-$CH_2$; 3,8 ppm (t), 2H, $CH_2$-O; 6,0 ppm (t), 1H, CH=C; 6,3 à 7,3 ppm (m), 14H, Ø.

Dérivé 16 : 1,6 à 3,3 ppm (m), 16H, $CH_2$-$CH_2$-$CH_2$-N-$CH_2$-$CH_2$-N-$CH_2$-C-O; 4,0 ppm (t), 2H, $CH_2$-O; 6,0 ppm (t), 1H, CH=C; 6,7 à 7,4 ppm (m), 14H, Ø.

Dérivé 17 : 2,2 à 2,7 ppm (m), 14H, $CH_2$-N,$CH_2$-C=C; 4,0 ppm (t), 2H, $CH_2$-O; 6,0 ppm (t), 1H, CH=C; 6,8 à 7,4 ppm (m), 13H, Ø.

I - ESSAI DE TOXICITE

La toxicité des composés de l'invention a été déterminée par le test ci-après :

- détermination de la dose létale 50 ($DL_{50}$) chez la souris

Le produit à tester a été administré par voie intrapéritonéale à des groupes de 5 souris mâles et 5 souris femelles, à raison de 0,1 ml pour 10 g de poids corporel.

Les doses suivantes ont été utilisées :

100 - 150 - 200 - 300 - 400 $mg.kg^{-1}$ I.P.

La $DL_{50}$ déterminée à partir de la mortalité observée est indiquée dans le tableau suivant ainsi que celle des composés connus comme antidépresseurs : l'AMINEPTINE et l'IMIPRAMINE.

TABLEAU II

| N° | $DL_{50}$ I.P. mg/kg | LIMITES FIDUCIELLES CALCUL SELON LITCHFIELD et WILCOXON |
|---|---|---|
| 1 | 231 | 195-274 |
| 2 | # 200 | - |
| 3 | # 250 | - |
| 4 | 386 | 279-453 |
| 5 | 314 | 234-421 |
| 6 | 247 | 206-296 |
| 7 | # 175 | - |
| 8 | 199 | 172-229 |
| AMINEPTINE | # 200 | - |
| IMIPRAMINE | # 115 | - |

## II - ESSAIS PHARMACOLOGIQUES

Les propriétés pharmacologiques des composés de l'invention on été déterminées en utilisant les tests ci-après :

### Protocoles d'essais

#### 1. Etude de la motilité spontanée

L'activité motrice des souris a été déterminée à l'aide de l'actimètre photoélectrique de Boissier et Simon.

Les souris sont placées par cinq dans une boîte fermée par un couvercle, et traversée par deux rayons lumineux perpendiculaires que les souris coupent en se déplaçant.

Ces déplacements sont comptabilisés par un compteur relevé 30 min et 1 h après l'administration du dérivé à tester.

#### 2. Comportement d'exploration

30 min après l'administration intrapéritonéale des dérivés selon l'invention, chaque souris est placée sur une planche à trous automatisée pendant 5 min, et on note, minute par minute, le nombre de trous explorés.

Une dose efficace 50 peut être calculée en fonction des résultats obtenus.

#### 3. Action myorelaxante (test de traction)

Ce test apprécie la présence ou l'absence de redressements d'une souris présentée par ses pattes antérieures à un fil métallique horizontal.

On note le nombre de souris qui ne peuvent accrocher au fil, une de leurs pattes postérieures avant 5 s.

Une dose efficace 50 peut être calculée en fonction des résultats obtenus.

#### 4. Température rectale

La température rectale des souris est mesurée à l'aide d'un thermomètre électrique Ellab. ctd 85.

On effectue une première lecture immédiatement avant l'injection du dérivé à tester.

La température est notée 30 min, 1, 2, 3 et 4 h après l'injection.

#### 5. Activité analgésique périphérique

Une douleur péritonéale de la souris est provoquée par injection intrapéritonéale de phénylbenzoquinone (P.B.Q.)

On recherche la diminution du syndrome douloureux caractérisé par une torsion abdominale à l'aide d'une injection du dérivé à tester 30 min avant l'administration de la P.B.Q.

La dose efficace 50 est calculée en fonction du pourcentage de diminution du syndrome douloureux par rapport aux témoins.

#### 6. Activité analgésique centrale

On recherche une augmentation du temps de passage sur une plaque chauffée à 60°C chez les souris traitées par le dérivé à tester, 30 min avant le début de l'essai.

La dose efficace 50 est calculée en fonction du pourcentage d'augmentation du temps passé sur la plaque chauffante (léchage des pattes ou éventuels sauts).

#### 7. Interaction avec le pentobarbital

On recherche une éventuelle augmentation du sommeil barbiturique en administrant par voie intrapéritonéale le dérivé à tester 5 min avant l'injection intrapéritonéale du pentobarbital (37,5 mg/kg).

Une dose efficace 50 peut être calculée en fonction des résultats obtenus.

#### 8. Interaction avec l'oxotrémorine

L'oxotrémorine étant un agoniste des récepteurs cholinergiques, on peut penser que les substances qui antagonisent les tremblements, l'hypothermie et les signes périphériques (salivation, pilo-érection) induits par ce produit sont des anticholinergiques.

Le dérivé à tester est administré 30 min avant l'injection intrapéritonéale de l'oxotrémorine.

#### 9. Test à la yohimbine

Le dérivé à tester est administré 30 min avant l'injection de yohimbine (25 mg/kg I.P.)

On compte le nombre de morts 24 h après l'injection de yohimbine.

#### 10. Test à l'apomophine

Le dérivé à tester est administré par voie intrapéritonéale 30 min avant l'injection de l'apomorphine.

L'apomorphine est injectée par voie sous-cutanée à raison de 16 mg/kg.

Les souris sont alors isolées dans des cages de petite dimension.

On note les redressements, les stéréotypies et la température rectale des animaux.

11. Test à la réserpine

Le dérivé à tester est administré par voie intrapéritonéale.

Les souris sont placées en cages individuelles.

30 min après l'injection du dérivé à tester, on injecte par voie intrapéritonéale la réserpine (2,5 mg/kg).

On note toutes les 30 min l'occlusion des paupières et on prend la température rectale 2 h, 3 h et 4 h après l'injection de la réserpine.

12. Test anticataleptique avec la P.C.P.Z.

On injecte le dérivé à tester et on place chaque rat dans des cages individuelles.

30 min après, on injecte 25 mg/kg de la P.C.P.Z., c'est-à-dire la prochloropérazine.

Toutes les 30 min, chaque rat est placé sur une feuille de papier-filtre et est soumis au croisement des pattes antérieures avec les pattes postérieures homolatérales.

13. Test de Porsolt

Le dérivé à tester est administré par voie intrapéritonéale 60 min avant le début de l'essai.

Les souris sont alors placées dans un bécher à moitié rempli d'eau pendant 6 min.

On calcule le temps de mouvement des animaux dans l'eau.

14. Test de la queue (Tail Suspension Test ou T.S.T.)

Un rongeur soumis à une situation désagréable et manifestement sans issue (accroché par la queue) a tendance à diminuer rapidement son activité motrice d'évasion.

L'appareil comprend deux unités d'accrochage, une unité centrale et un micro-ordinateur qui intègre les commandes de l'opérateur et les calculs statistiques.

Le dérivé à tester est administré par voie intrapéritonéale 30 min avant l'accrochage des souris.

La mesure des divers paramètres (temps d'immobilité, énergie totale, puissance des mouvements) s'effectue automatiquement pendant 6 min.

Résultats

Les résultats obtenus sont consignés dans le tableau III ci-après. Ces résultats montrent que les dérivés de l'invention présentent une activité sur le système nerveux central et en particulier des propriétés antidépressives.

Essais comparatifs

A titre de comparaison, on a effectué les tests ci-dessus avec l'AMINEPTINE, L'IMIPRAMINE et la MIANSERINE, produits connus pour leurs propriétés antidépressives. Les résultats obtenus figurent également dans le tableau III ci-après. Ces résultats montrent que les dérivés de l'invention et notamment le composé n°1 sont des antidépresseurs dont l'activité est supérieure à celle des antidépresseurs connus.

De plus le composé de l'exemple 1 a été testé pour son activité antisérotoninergique selon les tests ci-après:

Protocoles d'essais

1 - Mouvements saccadés de la tête (head-twitches) provoqués par le 5-hydroxytryptophane (5 H.T.P.)

Animaux : Souris mâles 25 ± 1 g

Au temps T-3h on a injecté par voie intrapéritonéale 100 mg/kg de Pargyline.

Au temps T-30 minutes on a injecté par voie intrapéritonéale le produit à tester ou de l'eau distillée (témoin).

Au temps T=0 on a injecté par voie intrapéritonéale du 5 H.T.P. à la dose de 4 mg/kg.

On a observé chez la souris tous les mouvements saccadés de la tête pendant 1 minute toutes les douze minutes

On a calculé le nombre moyen des mouvements et on les a comparés au lot témoin.

2 - Mouvements saccadés de la tête (head twitches) provoqués par la 5-méthoxydiméthyltryptamine (5.MeoDMT)

Aninaux : Souris mâles 25 ± 1 g

Au temps T-3 heures on a injecté par voie intrapéritonéale 100 mg/kg de Pargyline.

Au temps T-30 minutes on a injecté par voie intrapéritonéale du produit à étudier ou d'eau distillée (témoin)

Au temps T-0 on a injecté par voie intrapéritonéale du 5 MeoDMT à la dose de 4 mg/kg.

On a observé le nombre de mouvements saccadés de la tête.

On a calculé le nombre moyen de mouvements et on les a comparés au lot témoin.

3 - Erections peniennes provoquées par l'apomorphine

Les rats placés en cage transparente ont reçu par voie parentérale ou par voie orale le produit à essayer 30 minutes avant l'injection intrapéritonéale de 0,20 mg/kg d'apomorphine.

On a relevé le nombre d'érections péniennes pendant les 30 minutes qui suivent l'injection d'apomorphine.

L'étude statistique a été effectuée par rapport aux nombres d'érections des rats témoins.

4 - Test à la Tryptamine

La Tryptamine provoque des crises convulsives caractéristiques chez le rat.

Le produit à tester a été administré par voie intrapéritonéale 1 heure avant l'injection intraveineuse de Tryptamine (40 mg/kg)

On a observé si le produit peut diminuer l'intensité et/ou la durée de la crise convulsive.

Les calculs statistiques ont été effectués par rapport aux résultats observés chez le témoin.

5 - Test de Grahame Smith

Animaux : Rats mâles 200 ± 10 g

Au temps T-30 minutes le produit à tester a été administré par voie intrapéritonéale.

Au temps T=0 on a administré un I M A O par voie intrapéritonéale (Tranylcypromine 20 mg/kg).

Au temps + 20 minutes on a administré 250 mg/kg de Tryptophane (I.P.) Toutes les 30 minutes pendant 200 minutes, on a relevé la température des rats et on a noté par 0 à 1 les divers signes cliniques (agitation motrice - hypersalivation - catatonie de la queue -hyperesthésie - fouettement de la queue ...)

On a calculé la moyenne des températures, et celle des symptômes observés par rappport aux témoins.

Résultats Les résultats obtenus sont consignés dans le tableau IV ci-après. Ces résultats montrent que le composé de l'exemple 1 possède une activité antisérotoninergique.

TABLEAU III

| | Activité souris $mg.kg^{-1}$ I.P. | Comportement d'exploration souris $mg.kg^{-1}$ I.P. | Action myo-relaxante Test de traction souris $mg.kg^{-1}$ I.P. | Température rectale souris | Activité analgésique périphérique souris $DE_{50}$ $mg.kg^{-1}$ I.P. | Activité analgésique centrale souris $mg.kg^{-1}$ I.P. | Narcose barbiturique souris | Interaction avec l'oxotrémorine souris | Toxicité à la yohimbine souris $DE_{50}$ $mg.kg^{-1}$ I.P. | Antagonisme des symptômes APOMORPHINE 16 $mg.kg^{-1}$ S.C. souris $mg.kg^{-1}$ I.P. | Antagonisme des symptômes RESERPINE souris $mg.kg^{-1}$ I.P. | Interaction avec la P.C.P.Z rats $mg.kg^{-1}$ I.P. | Test de Porsolt souris $mg.kg^{-1}$ I.P. | T.S.T. souris $mg.kg^{-1}$ I.P. |
|---|---|---|---|---|---|---|---|---|---|---|---|---|---|---|
| 1 | Aucune modification jusqu'à 25 | Aucune modification jusqu'à 25 | Aucune modification jusqu'à 25 | Hypothermie fugace | 1,50 | Sans activité jusqu'à 12,5 | Aucune augmentation du sommeil barbiturique | Aucun antagonisme | 31 | Antagonise l'hypothermie à 6,25 | Antagonise l'hypothermie à 3,12 | Antagonise la catalepsie à 25 | Actif jusqu'à 3,12 | Actif jusqu'à 3,12 |
| 2 | - | " | - | Aucune modification | - | Sans activité | - | " | Augmentation à partir de 6,25 | Aucun antagonisme | Antagonisme quasi nul | - | Actif à 25 | Actif à 25 |
| 3 | - | " | - | " | - | " | Augmentation du sommeil barbiturique à 25,0 | " | Importante augmentation à 12,5 | " | Très faible antagonisme | - | Actif à 12,5 | Actif à 25 |
| 4 | Augmentation | " | " | " | 1,50 | " | Aucune augmentation | " | Faible augmentation | " | Antagonise l'hypothermie à 12,5 | Antagonise la catalepsie à 12,5 et 25 | Inactif | Inactif |
| 5 | Aucune modification jusqu'à 25 | " | " | " | - | " | Augmentation du sommeil barbiturique à 25 $mg.kg^{-1}$ | " | Aucune augmentation | " | " | - | " | " |
| 6 | Diminution nette | " | " | - | Faible activité | Sans activité jusqu'à 25 | Légère augmentation du sommeil barbiturique | " | 25 | " | " | Aucun antagonisme à 25 | " | " |

TABLEAU III (suite)

| | Activité souris mg.kg$^{-1}$ I.P. | Comportement d'exploration souris mg.kg$^{-1}$ I.P. | Action myo-relaxante Test de traction souris mg.kg$^{-1}$ I.P. | Température rectale souris | Activité analgésique périphérique souris $DE_{50}$ mg.kg$^{-1}$ I.P. | Activité analgésique centrale souris mg.kg$^{-1}$ I.P. | Narcose barbiturique souris | Interaction avec l'oxotrémorine souris | Toxicité à la yohimbine souris $DE_{50}$ mg.kg$^{-1}$ I.P. | Antagonisme des symptômes APOMORPHINE 16 mg.kg$^{-1}$ S.C. souris mg.kg$^{-1}$ I.P. | Antagonisme des symptômes RESERPINE souris mg.kg$^{-1}$ I.P. | Interaction avec la P.C.P.Z rats mg.kg$^{-1}$ I.P. | Test de Porsolt souris mg.kg$^{-1}$ I.P. | T.S.T. souris mg.kg$^{-1}$ I.P. |
|---|---|---|---|---|---|---|---|---|---|---|---|---|---|---|
| 7 | $DE_{50}$ 7,5 mg.kg$^{-1}$ | Aucune modification jusqu'à 25 | Aucune modification jusqu'à 25 | Hypothermie à 25 | 0 | - | Augmentation du sommeil barbiturique | Aucun antagonisme | >25 | Aucun antagonisme | Aucun antagonisme | Aucun antagonisme | Inactif | - |
| 8 | Diminution à 25 | " | " | Hypothermie fugace | 6,25 | - | Faible augmentation du sommeil barbiturique | " | >25 | " | Antagonise l'hypothermie à 12,5 | Antagonise la catalepsie à 25 | Inactif | - |
| 14 | Diminution | Aucune modification | Aucune modification | Aucune modification | 2,5 | Significatif à 25 | Augmentation du sommeil barbiturique à 4,9 | Aucun antagonisme | > 25 | Antagonise l'hypothermie | Antagonise l'hypothermie | Quasi inactif | Actif à 25,0 | Actif à 25,0 |
| 16 | Augmentation importante | " | " | " | 4,5 | Aucune activité | Aucune augmentation | Antagonise l'hypothermie | Peu actif | Aucun antagonisme | Aucun antagonisme | Inactif | Inactif | Augmentation de l'énergie et puissance des mouvements |
| 17 | Augmentation à 7,9 | " | Actif à 25 | " | 5,2 | " | " | Aucun antagonisme | >25 | Antagonise l'hypothermie à 6,25 | Antatonise à dose faible l'hypothermie | Léger antagonisme | Actif à 6,25 | Très actif à 1,58 |

TABLEAU III (suite)

| PRODUITS | Activité souris mg.$kg^{-1}$ I.P. | Comportement d'exploration souris mg.$kg^{-1}$ I.P. | Action myo-relaxante Test de traction souris mg.$kg^{-1}$ I.P. | Température rectale souris | Activité anal-gésique péri-phérique souris $DE_{50}$ mg/$kg^{-1}$ I.P. | Activité anal-gésique cen-trale souris mg.$kg^{-1}$ I.P. | Interaction avec l'oxo-trémorine souris | Toxicité à la yohimbine souris $DE_{50}$ mg.$kg^{-1}$ I.P. | Antagonisme des symptômes APOMORPHINE 16 mg.$kg^{-1}$ S.C. souris mg.$kg^{-1}$ I.P. | Antagonisme des symptômes RESERPINE souris | Interaction avec la P.C.P.Z rats mg.$kg^{-1}$ I.P. | Test de Porsolt souris mg.$kg^{-1}$ I.P. | T.S.T. souris mg.$kg^{-1}$ I.P. |
|---|---|---|---|---|---|---|---|---|---|---|---|---|---|
| AMINEPTINE | Augmente l'acti-vité | Aucune modifica-tion | Aucune action | Hypo-thermie fugace | - | - | Aucun antago-nisme | Augmenta-tion à partir de 10 | Aucun antago-nisme | Antago-nise l'hypo-thermie | - | Actif à 6,25 | Actif à 12,5 |
| IMIPRAMINE | Augmente l'acti-vité à 15mg/kg | " | " | " | - | - | Diminue les tremble-ments | Augmenta-tion à partir de 15 | Antagonise l'hypo-thermie | " | Anta-gonise à 15 | Actif à 8 | Actif à 8 |
| MIANSERINE | - | - | - | - | - | - | - | - | - | - | - | Actif à 16 | Actif à 3,13 |

Tableau IV

ACTIVITE ANTISEROTONINERGIQUE

| | Antagonisme des Head-Twitches induits par | | Antagonisme des érections péniennes et des baillements (rat) | Test à la tryptamine (rat) | Test de Grahame Smith (rat) |
|---|---|---|---|---|---|
| | 5-HTP | 5-MeODMT | | | |
| Composé 1(IP) | ↘dès 18,8mg/kg | ↘ dès 6,25mg/kg | angatonisme dès 6,25 mg/kg | actif dès 50mg/kg | ↘Hyperthermie et signes cliniques |
| Composé 1(PO) | nt | nt | nt | nt | ↘à 37,5mg/Kg |
| Miansérine(IP) | nt | nt | nt | actif à 6,0mg/kg | |

nt = non testé

## Revendications

1. Dérivés de la 1-[(1,1-diphényl)-1-alcényl]-pipérazine répondant à la formule générale I :

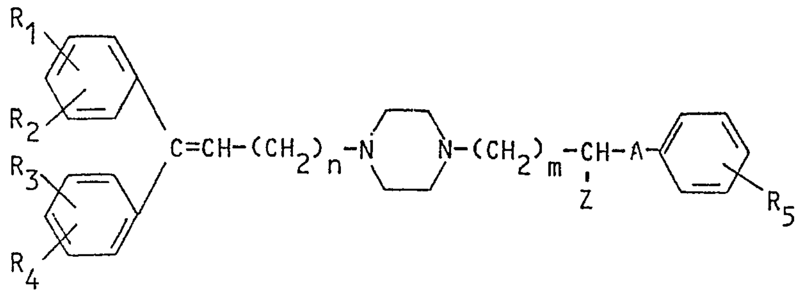

(I)

dans laquelle
$R_1$, $R_2$, $R_3$, $R_4$ et $R_5$, identiques ou différents, représentent un atome d'hydrogène, un atome d'halogène, un groupe alkyle en $C_1$-$C_6$, un groupe alcényle en $C_1$-$C_6$, un groupe alcoxy en $C_1$-$C_6$ ou le groupe trifluorométhyle;
n est un nombre entier compris entre 1 et 3 ;
m est un nombre entier de 0 à 3;
Z représente un atome d'hydrogène, un groupe alkyle en $C_1$-$C_6$ ou un groupe aryle de formule

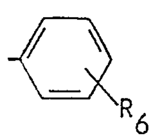

dans laquelle $R_6$ a la même signification que $R_1$, $R_2$, $R_3$ ou $R_4$;
A est un atome d'oxygène ou un groupe $-\underset{\underset{O}{\|}}{C}-$ ;
et leurs sels pharmaceutiquement acceptables.

2. Dérivé selon la revendication 1, caractérisé en ce qu'il est la 1-[(1,1-diphényl)-1-butène-4-yl]--4-[1-(2-(-4-fluorophénoxy)éthyl]-pipérazine

3. Procédé pour l'obtention des dérivés selon la revendication 1, caractérisé en ce qu'il consiste à faire réagir un dérivé de formule générale II :

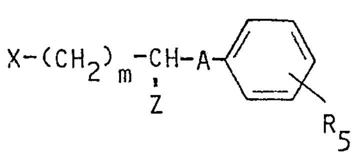

(II)

dans laquelle Z, m, A sont tels que définis ci-dessus, et X est un groupe tosyle ou un atome d'halogène (par exemple le brome ou le chlore),
avec une pipérazine de formule générale III :

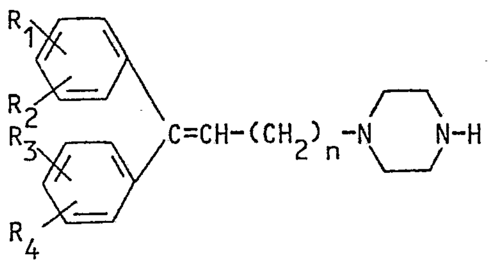

(III)

dans laquelle $R_1$, $R_2$, $R_3$, $R_4$ et n sont tels que définis précédemment, en présence d'iodure de sodium ou de potassium et d'un équivalent de carbonate de sodium ou de potassium et à transformer les composés obtenus en leurs sels pharmaceutiquement acceptables.

4. Compositions pharmaceutiques, caractérisées en ce qu'elles contiennent à titre d'ingrédient actif un dérivé selon la revendication 1 en combinaison avec un véhicule pharmaceutiquement acceptable.

5. Utilisation des dérivés de la 1-[(1,1-diphényl)-1-alcényl]-pipérazine répondant à la formule générale I :

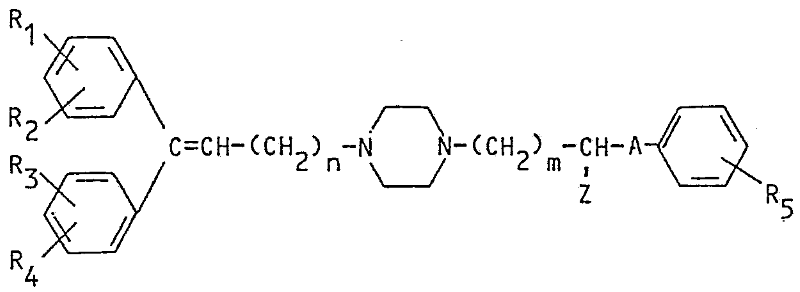

(I)

dans laquelle
$R_1$, $R_2$, $R_3$, $R_4$ et $R_5$, identiques ou différents, représentent un atome d'hydrogène, un atome d'halogène, un groupe alkyle en $C_1$-$C_6$, un groupe alcényle en $C_1$-$C_6$, un groupe alcoxy en $C_1$-$C_6$ ou le groupe trifluorométhyle;
n est un nombre entier compris entre 1 et 3;
m est un nombre entier de 0 à 3;
Z représente un atome d'hydrogène, un groupe alkyle en $C_1$-$C_6$ ou un groupe aryle de formule

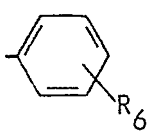

dans laquelle $R_6$ a la même signification que $R_1$, $R_2$, $R_3$ ou $R_4$;
A est un atome d'oxygène ou un groupe $-\underset{\underset{O}{\parallel}}{C}-$ ;
et leurs sels pharmaceutiquement acceptables à la fabrication de médicaments utiles sur le système nerveux central.

**Revendications pour les Etats contractants suivants : ES,GR**

1. Procédé pour l'obtention de dérivés de la 1-[(1,1-diphényl)-1-alcényl]-pipérazine répondant à la formule générale I :

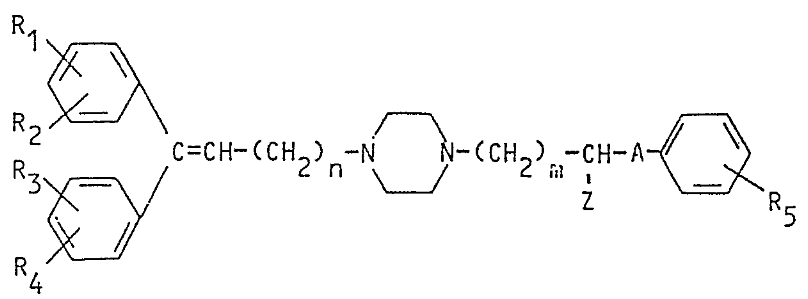

(I)

dans laquelle

$R_1$, $R_2$, $R_3$, $R_4$ et $R_5$, identiques ou différents, représentent un atome d'hydrogène, un atome d'halogène, un groupe alkyle en $C_1$-$C_6$, un groupe alcényle en $C_1$-$C_6$, un groupe alcoxy en $C_1$-$C_6$ ou le groupe trifluorométhyle;

n est un nombre entier compris entre 1 et 3;

m est un nombre entier de 0 à 3;

Z représente un atome d'hydrogène, un groupe alkyle en $C_1$-$C_6$ ou un groupe aryle de formule

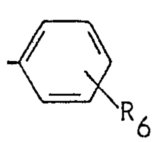

dans laquelle $R_6$ a la même signification que $R_1$, $R_2$, $R_3$ ou $R_4$;

A est un atome d'oxygène ou un groupe $-\underset{\underset{O}{\|}}{C}-$ ;

et leurs sels pharmaceutiquement acceptables, caractérisé en ce qu'il consiste à faire réagir un dérivé de formule générale II :

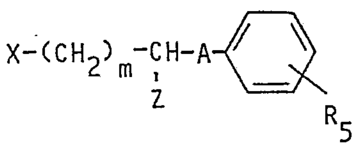

(II)

dans laquelle Z, m, A sont tels que définis ci-dessus, et X est un groupe tosyle ou un atome d'halogène (par exemple le brome ou le chlore), avec une pipérazine de formule générale III :

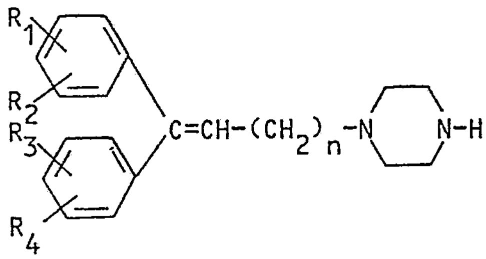

(III)

dans laquelle $R_1$, $R_2$, $R_3$, $R_4$ et n sont tels que définis précédemment, en présence d'iodure de sodium ou de potassium et d'un équivalent de carbonate de sodium ou de potassium et à transformer les composés obtenus en leurs sels pharmaceutiquement acceptables.

2. Procédé selon la revendication 1, caractérisé en ce que le dérivé de formule III est obtenu par réaction d'un dérivé de formule générale IV

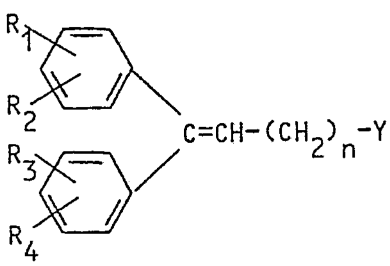

(IV)

dans laquelle $R_1$, $R_2$, $R_3$, $R_4$ et n sont tels que définis ci-dessus, et Y est un atome de brome ou de chlore, avec la pipérazine anhydre de formule V :

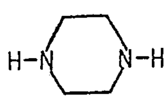

(V)

par chauffage au reflux dans un solvant approprié.

3. Procédé selon la revendication 1, caractérisé en ce que la pipérazine de formule III est la 1-(1,1-diphényl-1-butène-4-yl)-pipérazine et en ce que le composé de formule II est le 2-bromo-1-(4-fluorophénoxy)-éthane, le dérivé obtenu étant la 1-[(1,1-diphényl)-1-butène-4-yl]-4-[1-(2-(4-fluorophénoxy)éthyl]pipérazine.

4. Utilisation des dérivés de la 1-[(1,1-diphényl)-1-alcényl]-pipérazine répondant à la formule générale I :

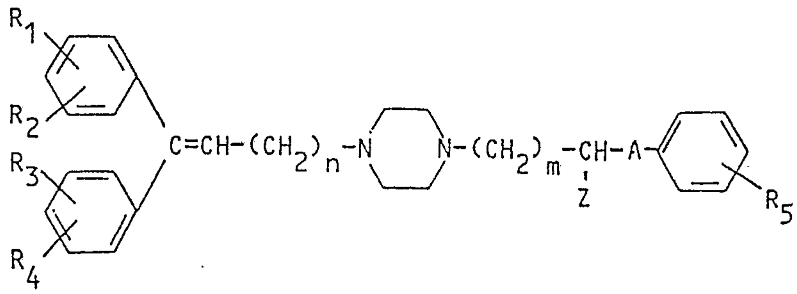

(I)

dans laquelle

$R_1$, $R_2$, $R_3$, $R_4$ et $R_5$, identiques ou différents, représentent un atome d'hydrogène, un atome d'halogène, un groupe alkyle en $C_1$-$C_6$, un groupe alcényle en $C_1$-$C_6$, un groupe alcoxy en $C_1$-$C_6$ ou le groupe trifluorométhyle;

n est un nombre entier compris entre 1 et 3;

m est un nombre entier de 0 à 3;

Z représente un atome d'hydrogène, un groupe alkyle en $C_1$-$C_6$ ou un groupe aryle de formule

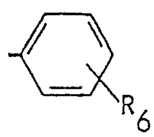

dans laquelle $R_6$ a la même signification que $R_1$, $R_2$, $R_3$ ou $R_4$;

A est un atome d'oxygène ou un groupe $-\underset{\underset{O}{\|}}{C}-$ ;

et leurs sels pharmaceutiquement acceptables à la préparation de médicaments utiles sur le système nerveux central.

Office européen des brevets

# RAPPORT DE RECHERCHE EUROPEENNE

Numero de la demande

EP 88 40 0903

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | JOURNAL OF MEDICINAL CHEMISTRY, vol. 15, no. 6, juin 1972; pages 665-673; C. KAISER et al.: "Analogs of phenothiazines. 4. Effect of structure upon neuropharmacological activity of some chlorpromazine analogs of the diphenylmethane type" * Page 665; table I * --- | 1-5 | C 07 D 295/08<br>C 07 D 295/10<br>A 61 K 31/495 |
| Y | CHEMICAL ABSTRACTS, vol. 107, no. 3, 20 juillet 1987, résumé no. 17884s, Columbus, Ohio, US; & ES-A-524 680 (SOCIEDAD ESPANOLA DE ESPECIALIDADES FARMACO-TERAPEUTICAS S.A.) 16-12-1984 * Résumé * ----- | 1,4,5 | |
| | | | **DOMAINES TECHNIQUES RECHERCHES (Int. Cl.4)** |
| | | | C 07 D 295/00<br>A 61 K 31/00 |

Le présent rapport a été établi pour toutes les revendications

| Lieu de la recherche | Date d'achèvement de la recherche | Examinateur |
|---|---|---|
| LA HAYE | 01-08-1988 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)